# EUROPEAN PATENT SPECIFICATION

(11) **EP 1 805 126 B1**
(45) Date of publication and mention of the grant of the patent: **24.11.2010**
(21) Application number: 05797104.6
(22) Date of filing: 30.09.2005
(51) Int. Cl.: C07C 51/02, C07C 59/19

(54) **METHOD FOR PRODUCING PYRUVIC ACID**
VERFAHREN ZUR HERSTELLUNG VON BRENZTRAUBENSÄURE
PROCEDE DE PRODUCTION DE L'ACIDE PYRUVIQUE

(30) Priority: 01.10.2004 NO 20044218
(43) Date of publication of application: 11.07.2007
(73) Proprietor: GE HEALTHCARE AS, 0401 Oslo (NO)
(72) Inventor: ROLANDSGAARD, Marit, N-0401 Oslo (NO); SANDOSHAM, Jessie, N-0401 Oslo (NO); THOMASSEN, Terje, N-0401 Oslo (NO); GRACE, David, N-0401 Oslo (NO)
(74) Representative: Wulff, Marianne Weiby
(86) International application number: PCT/NO2005/000363
(87) International publication number: WO 2006/038811

(56) References cited:
- US-B1- 6 232 497
- US-B1- 6 670 505
- U. HOLLSTEIN, D. MOCK, R. SIBBITT, U. ROISCH AND F. LINGENS: "Synthesis of Shikimic-6-13C acid" JOURNAL OF RADIOLABELLED COMPOUNDS AND RADIOPHARMACEUTICALS, vol. 17, no. 2, 1980, pages 289-296, XP008057202
- J. E. THIRKETTLE, J. E. BALDWIN, J. EDWARDS, J. P. GRIFFIN AND C. J. SCHOLFIELD: "The origin of the beta-lactam carbons of clavulinic acid" CHEM. COMMUN., 1997, pages 1025-1026, XP002358982

## Description

The invention relates to a method for the production of pyruvic acid.

Pyruvic acid and salts thereof are important intermediates found in the pathway of carbohydrate metabolism within the living body. In order to gain insight in this metabolic pathway, isotopically enriched pyruvic acid or salts thereof like for instance ¹³C- or ¹⁴C-pyruvic acid can be used allowing the detection of metabolites generated in the living body by either ¹³C-NMR or radioactive detection methods.

Several methods for the production of pyruvic acid are known in the art which can be grossly divided into methods involving the use of microorganisms or enzymes and chemical synthesis.

An example of an enzyme based method for the production of pyruvic acid is the enzymatic oxidation of lactic acid, as for instance described in WO-A-95/00656. Enzymatic oxidation often results in byproducts as hydrogenperoxide is produced during said enzymatic oxidation. Further, an upscale of enzymatic processes to an industrial process level is often problematic or impossible. Examples for methods involving the use of microorganisms for the production of pyruvic acid are for instance described in EP-A-313 850. A disadvantage of these microbiological production processes is that it is often difficult and time consuming to separate, isolate and purify pyruvic acid from the complex reaction mixtures, e.g. form complex fermentation broths.

Examples of chemical synthesis for the production of pyruvic acid are largely based on the oxidation of various starting materials like propylene glycol (as described in EP-A-337 246), hydroxyacetone (described in US-A-4 247 716) or lactic acid (see for instance JP-A-8183753). However, for the production of isotopically enriched ¹⁴C- or ¹³C-pyruvic acid, the use of a commercially available isotopically enriched starting material or an isotopically enriched starting material that is obtainable by a straightforward chemical synthesis is greatly preferred.

S. Anker, J. Biol. Chem 176, 1948, 1333-1335 describes the synthesis of 2-¹⁴C-enriched pyruvic acid which is obtained by using to synthesizing 1-¹⁴C-potassium acetate from ¹⁴C-enriched barium carbonate, converting it to 1-¹⁴C-acetyl bromide and subsequently reaction with cuprous cyanide to 1-¹⁴C-acetyl cyanide. The acetyl cyanide is reacted to 2-¹⁴C-pyruvamide which is then hydrolysed to 2-¹⁴C-pyruvic acid. Using isotopically enriched cuprous cyanide and acetyl bromide allows the synthesis of a pyruvic acid that is isotopically enriched at the C1-atom.

U. Hollstein et al., Journal of Labelled Compounds and Radiopharmaceuticals Vol. XVII, No. 2, 1980, pages 289-296 discloses a method for the preparation of ¹³C₂-pyruvic acid comprising the transformation of ¹³C₂-acetic acid into ¹³C₂-acetyl bromide and its further reaction with CuCN to afford the amide derivative (pyruvamdie) which upon hydrolysis with HCl in a mixture of ether and water yields ¹³C₂-pyruvic acid.

J.E. Thirkettle et al., Chem. Commun. 1997, pages 1025-1026 discloses a method for the preparation of ¹³C-enriched pyruvic acid analogous to the one disclosed by Hollstein et al. ¹³C₁-acetic acid is used as starting material and Cu¹³CN as reagent which results in pyruvic acid which is enriched at the C1 and C2-position. The conversion of the ¹³C-enriched pyruvamide to pyruvic acid takes place upon reaction with an acidic resin.

As ¹³C-enriched sodium pyruvate is commercially available, protonation of the pyruvate is a straightforward way to obtain ¹³C-enriched pyruvic acid. US-B-6 232 497 describes the protonation of sodium pyruvate with 70% sulphuric acid in ethyl acetate.

We have now surprisingly found a method to produce pyruvic acid in excellent purity and high yield, said method comprising reacting a salt of pyruvic acid with hydrochloric acid in the presence of a solvent from the group consisting of polar aprotic nitriles and ketones, isolating the crude pyruvic acid obtained and optionally purifying said crude pyruvic acid.

Hence the invention provides a method to produce pyruvic acid comprising reacting a salt of pyruvic acid with hydrochloric acid in the presence of a solvent or solvent mixture from the group consisting of polar aprotic nitriles and ketones, isolating the crude pyruvic acid obtained and optionally purifying said crude pyruvic acid.

In the following the term "salts of pyruvic acid" and pyruvates" are used interchangeably. In the method according to the invention a salt of pyruvic acid is used as a starting material. Preferred salts of pyruvic acid are alkali metal pyruvates, alkaline earth metal pyruvates or pyruvates comprising an organic cation, more preferred pyruvates are alkaline metal pyruvates containing as cation one from the group comprising Li, Na, K, Rb and Cs with sodium pyruvate and potassium pyruvate being the most preferred ones.

In a preferred embodiment, isotopically enriched pyruvates are used in the method of the invention, preferably ¹³C- and ¹⁴C-enriched pyruvates, and more preferably ¹³C-enriched pyruvates.

The isotopic enrichment of the pyruvate used in the method of the invention is preferably at least 75%, more preferably at least 80% and especially preferably at least 90%, an isotopic enrichment of over 90% being most preferred. Ideally, the enrichment is 100%. Pyruvate may be isotopically enriched at the C1-, the C-2 or the C3-position, at the C1- and the C2-position, at the C1- and the C3-position, at the C2- and the C3-position or at the C1-, C2- and C3-position; the C1-position being the preferred one.

The hydrochloric acid used in the method of the invention is preferably 1 to 12.1 M more preferably 8 to 12.1 M. Most preferably a concentrated hydrochloric acid, i.e. 12.1 M, is used as this minimizes the water content in the crude pyruvic acid. The ratio of pyruvate to hydrochloric acid can be varied within some limits but it has proved to be especially advantageous if pyruvate and hydrochloric acid are reacted in stoichiometric or approximately stoichiometric ratios. If approximately stoichiometric ratios are used, a slight excess of hydrochloric acid is preferred.

According to method of the invention, a solvent or solvent mixture from the group consisting of polar aprotic nitriles and ketones is used. In a preferred embodiment, the solvent is acetone, acetylacetone, methyl ethyl ketone, methyl isobutyl ketone, acetonitrile or valeronitrile or a mixture thereof. More preferably the solvent is acetone or acetonitrile or a mixture thereof.

The method according to the invention can be carried out at a broad temperature range. As the reaction is exothermic, the hydrochloric acid is preferably gradually added to the pyruvate/solvent mixture and the temperature is monitored. The temperature upon addition is preferably below ambient temperature, preferably of from 10 °C to 0 °C which for instance may be achieved by cooling the reaction vessel in an ice bath. After addition of the hydrochloric acid, the reaction is conveniently run at temperatures around ambient temperature, preferably in a temperature range of from 15 °C to 40 °C.

In a preferred embodiment, the method of the invention is carried out by preparing a solution or suspension from sodium or potassium pyruvate and the solvent or solvent mixture. Said solution or suspension is cooled to about 0 °C and the hydrochloric acid is added gradually. The reaction mixture is warmed up to ambient temperature after complete addition of the hydrochloric acid and stirred for about 1 to 2 hours. After completion of the reaction, precipitated sodium or potassium chloride is filtered off.

The crude pyruvic acid obtained usually contains water and salt. It may be isolated by for instance filtrating off the salt resulted from the reaction and/or evaporating the solvent or solvent mixture used in the reaction, e.g. by evaporation under reduced pressure and/or elevated temperature on a rotary evaporator.

In a preferred embodiment of the method of the invention, the isolated crude pyruvic is further purified. The choice of the purification method is among other things dependent on the amount of water present in the crude pyruvic acid, which in turn is dependent on the concentration of the hydrochloric acid used in the reaction.

If a hydrochloric acid with a relative high water content was used in the reaction, purification is preferably carried out by extraction of the pyruvic acid, optionally followed by a final distillation of the purified pyruvic acid obtained. To perform the extraction, the crude pyruvic acid is preferably dissolved in a solvent or solvent mixture containing a solvent selected from the group consisting of ethers, more preferably diethyl ether, di-tert.-butyl ether or dimethyl ether, most preferably diethyl ether and the water and/or salt that separates is removed, e.g. by separating the organic and the aqueous layer formed and/or filtration of the salt. Subsequently, the solvent or solvent mixture used is removed, preferably by evaporation, e.g. by evaporation at elevated temperature and/or reduced pressure on a rotary evaporator. Optionally, the purified pyruvic acid obtained from the extraction can be further purified by distillation under vacuum.

If a concentrated hydrochloric acid was used in the reaction, purification is preferably carried out by precipitation of the remaining salt, e.g. sodium chloride if sodium pyruvate was used as starting material. To perform the precipitation, a solvent or solvent mixture form the group consisting of ether, ester or ketone is added to the crude pyruvic acid, more preferably ether and ketone, most preferably acetone is added to promote the precipitation and the precipitated salt is filtered off. Subsequently, the solvent or solvent mixture used is removed, preferably by evaporation, e.g. by evaporation at elevated temperature and/or reduced pressure on a rotary evaporator. Optionally, the purified pyruvic acid obtained from the purification can be further purified by distillation under vacuum.

The purity of the pyruvic acid obtained may be controlled by various analytical methods like for instance NMR to confirm the structure and determine purity of the product, HPLC to determine purity and residual solvent by GC.

In a preferred embodiment, ¹³C-enriched pyruvate is used in the method of the invention. Several methods for the synthesis of ¹³C-pyruvate are known in the art. In a preferred embodiment, ¹³C-pyrovate is used in the method of the invention that is synthesised from 2-methyl-1,3-dithian. The dithian is metallated and reacted with CO₂ (Seebach et al., Journal of Organic Chemistry 40(2), 1975, 231-237). The carbonyl function is subsequently liberated by use of conventional methods described in the literature, for instance by Corey et al., J. Org. Chem. 36, 1971, 3553-3560. By using ¹³C-enriched CO₂, ¹³C-pyruvate which is enriched at the C1-position is obtained. The use of 2-methyl-1,3-dithian which is ¹³C-enriched at the methyl-group allows for the preparation of ¹³C-pyruvate which is enriched at the C3-position. Using ¹³C-enrichcd CO₂ and 2-methyl-1,3-dithian which is ¹³C-enriched at the methyl-group leads to ¹³C-pyruvate which is enriched at the C1 - and C3-postion.

Thus, in a preferred method of the invention ¹³C-enriched pyruvate is used that is synthesised by reacting 2-methyl-1,3-dithian or 2-methyl-1,3-dithian which is ¹³C-enriched at the methyl-group with optionally ¹³C-enriched CO₂, wherein at least the CO₂ or the 2-methyl-1,3-dithian is a ¹³C-enriched compound. In a further preferred embodiment of the method of the invention ¹³C-enriehed pyruvate is used that is enriched at the C1-position and synthesised by reacting 2-methyl-1,3-dithian with ¹³C-enriched CO₂..

In another preferred embodiment, ¹³C-pyruvate is used in the method of the invention which is synthesised by converting acetic acid into acetyl bromide and subsequently reacting the acetyl bromide with Cu¹³CN. The nitril obtained is converted into ¹³C-pyruvate isotopically enriched at the C1-position via the amide. To obtain ¹³C-pyruvate that is isotopically enriched at the C2-position, the synthesis is carried out using ¹³C-enriched acetic acid which can be synthesised as described in S. Anker, J. Biol. Chem 176, 1948, 1333-1335. To obtain ¹³C-pyruvate that is isotopically enriched at the C1-and the C2-position, ¹³C-enriched acetic acid is converted into ¹³C-enriched acetyl bromide and subsequently reacted with Cu¹³CN.

Thus, in another preferred method of the invention ¹³C-enriched pyruvate is used that is synthesised by converting optionally ¹³C-enriched acetic acid into acetyl bromide and reacting the acetyl bromide with optionally ¹³C-enriched CuCN, wherein at least the acetic acid or the CuCN is a ¹³C-enriched compound. In a further preferred embodiment of the method of the invention ¹³C-enriched pyruvate is used that is enriched at the C1-position and synthesised by converting acetic acid into acetyl bromide and reacting the acetyl bromide with ¹³C-enriched CuCN.

### Examples

### Example 1

¹³C₁-labelled sodium pyruvate (300 g) was suspended in acetonitrile (600 mL) in a reaction vessel in an ice-bath. Concentrated hydrochloric acid (223 mL, 12.1 M) was gradually added under stirring over a period of 1 hour. The ice-bath was removed after the addition had been completed and the suspension was stirred at ambient temperature for additional 2 hours. The precipitated sodium chloride was removed by filtration and the filtrate was evaporated to give the crude ¹³C₁-pyruvic acid (245 g).

Acetone (850 mL) and MgSO₄ (40 g) were added to the crude pyruvic acid to precipitate the residual sodium chloride and the precipitated salt was removed by filtration. The filtrate was evaporated and ¹³C₁-pyruvic acid isolated as a yellow oil. Purity = 99 % (NMR). Yield = 223 g (93 %); yield of pure pyruvic acid, i.e. corrected for salt/water = 86 %.

### Example 2

Sodium pyruvate was suspended in acetonitrile (2.4 mL/g sodium pyruvate) in a reaction vessel in an ice-bath. Concentrated hydrochloric acid (1 mole equiv., 0.745 mL/g sodium pyruvate, 12.1 M) was gradually added under stirring over a period of 1 hour. The ice-bath was removed after completed addition and the suspension was stirred at ambient temperature for approx. 2 hours. The precipitated sodium chloride was removed by filtration and the filtrate was evaporated to give the crude pyruvic acid (0.95-1.2 g/g sodium pyruvate)

Acetone (4 mL/g sodium pyruvate) was added to the crude product to precipitate remaining sodium chloride and MgSO₄ (0.15 g/g sodium pyruvate) was added to lower the water content. The solution was left in the fridge for one hour, before precipitated sodium chloride and MgSO₄ was removed by filtration. The filtrate was evaporated until water level was below 6 % w/w. Pyruvic acid was isolated as a pale yellow oil.

The results are shown in Table 1. Purity of pyruvic acid was determined by HPLC. Purity corrected means purity of pyruvic acid corrected for salt and water, the corrected purity was determined by HPLC as well. Yield means yield of pure pyruvic acid, i.e. corrected for salt and water.

**Table 1: Data from the production of ¹²C₁-pyruvic acid and ¹³C₁-pyruvic acid**

| **Isotope** | **Starting material (g)** | **Purity (HPLC) (%)** | **Purity corrected (HPLC) (%)** | **Yield corrected (%)** |
|---|---|---|---|---|
| ¹³C | 115 | 99.1 | 93.3 | 86.9 |
| ¹³C | 250 | 99.9 | 96.4 | 76.2 |
| ¹³C | 310 | 99.3 | 93.8 | 88.0 |
| ¹²C | 360 | 99.2 | 97.0 | 85.5 |
| ¹²C | 360 | 99.2 | 96.2 | 90.6 |
| ¹²C | 360 | 99.1 | 97.2 | 85.0 |
| ¹²C | 500 | 98.7 | 95.4 | 83.1 |

## Claims

1. Method for the production of pyruvic acid comprising reacting a salt of pyruvic acid with hydrochloric acid in the presence of a solvent or solvent mixture from the group consisting of polar, aprotic nitriles and ketones, isolating the crude pyruvic acid obtained and optionally purifying said crude pyruvic acid.

2. Method according to claim 1 wherein the solvent is acetone, acetylacetone, methyl ethyl ketone, methyl isobutyl ketone, acetonitrile or valeronitrile or a mixture thereof.

3. Method according to claims 1 to 2 wherein the solvent is acetone or acetonitrile or a mixture thereof.

4. Method according to claims 1 to 3 wherein the crude pyruvic acid is isolated by filtrating the salt resulted from the reaction and/or evaporating the solvent or solvent mixture used.

5. Method according to claims 1 to 4 wherein the crude pyruvic acid is purified.

6. Method according to claim 5 wherein the purification is carried out by extraction of the pyruvic acid, optionally followed by a distillation of the purified pyruvic acid

7. Method according to claim 5 wherein the purification is carried out by precipitation of the salt remained in the crude pyruvic acid, optionally followed by a distillation of the purified pyruvic acid.

8. Method according to claim 7 wherein said precipitation is promoted by adding a solvent or solvent mixture from the group consisting of ether, ester or ketone, preferably ether and ketone, most preferably acetone.

9. Method according to claims 1 to 8 wherein said salt of pyruvic acid is an alkali metal salt or alkaline earth metal salt, preferably sodium or potassium pyruvate.

10. Method according to claims 1 to 9 wherein said salt is a ¹³C-enriched pyruvate which is isotopically enriched at the C1-, the C2 or the C3 position.

11. Method according to claim 10 wherein the ¹³C-enriched pyruvate is synthesised by metallating 2-methyl-1,3-dithian or 2-methyl-1,3-dithian which is ¹³C-enriohed at the methyl group and reacting it with optionally ¹³C-enriched CO₂, wherein at least the CO₂ or the 2-methyl-1,3-dithian is a ¹³C-enriched compound and liberating the carbonyl function.

12. Method according to claim 11 wherein the ¹³C-enriched pyruvate is a ¹³C-pyruvate enriched at the C1-position that is synthesised by metallating 2-methyl-1,3-dithian, reacting it with ¹³C-enriched CO₂ and liberating the carbonyl function.

13. Method according to claim 10 wherein the ¹³C-enriched pyruvate is synthesised by converting optionally ¹³C-ennched acetic acid into acetyl bromide, reacting the acetyl bromide with optionally ¹³C-enriched CuCN, wherein at least the acetic acid or the CuCN is a ¹³C-enriched compound to obtain ¹³C-enriched pyruvamide which is then hydrolysed.

14. Method according to claim 10 wherein the ¹³C-enriched pyruvate is a ¹³C-pyruvate enriched at the C1-position that is synthesised by converting acetic acid into acetyl bromide,reacting the acetyl bromide with ¹³C-enriched CuCN to obtain ¹³C₁-pyruvamide which is then hydrolysed.

## Patentansprüche

1. Verfahren zur Herstellung von Brenztraubensäure, umfassend die Umsetzung eines Salzes der Brenztraubensäure mit Salzsäure in Gegenwart eines Lösungsmittels oder einer Lösungsmittelmischung aus der Gruppe bestehend aus polaren, aprotischen Nitrilen und Ketonen, das Isolieren der erhaltenen rohen Brenztraubensäure und die wahlweise Aufreinigung der rohen Brenztraubensäure.

2. Verfahren nach Anspruch 1, wobei das Lösungsmittel Aceton, Acetylaceton, Methylethylketon, Methylisobutylketon, Acetonitril oder Valeronitril oder eine Mischung daraus ist.

3. Verfahren nach Anspruch 1 oder 2, wobei das Lösungsmittel Aceton oder Acetonitril oder eine Mischung daraus ist.

4. Verfahren nach einem der Ansprüche 1 bis 3, wobei die rohe Brenztraubensäure durch Filtrieren des Salzes, welches aus der Umsetzung und/oder dem Verdampfen des verwendeten Lösungsmittels oder der Lösungsmittelmischung gebildet wird, isoliert wird.

5. Verfahren nach einem der Ansprüche 1 bis 4, wobei rohe Brenztraubensäure aufgereinigt wird.

6. Verfahren nach Anspruch 5, wobei die Aufreinigung durch Extraktion der Brenztraubensäure durchgeführt wird, wahlweise gefolgt von einer Destillation der aufgereinigten Brenztraubensäure.

7. Verfahren nach Anspruch 5, wobei die Aufreinigung durch Ausfällung des in der rohen Brenztraubensäure verbleibenden Salzes durchgeführt wird, wahlweise gefolgt von einer Destillation der aufgereinigten Brenztraubensäure.

8. Verfahren nach Anspruch 7, wobei die Ausfällung durch Zugabe eines Lösungsmittels oder Lösungsmittelmischung aus der Gruppe bestehend aus Ether, Ester oder Keton, bevorzugt Ester oder Keton, meist bevorzugt Aceton, beschleunigt wird.

9. Verfahren nach einem der Ansprüche 1 bis 8, wobei das Salz der Brenztraubensäure ein Alkalimetall- oder Erdalkalimetallsalz, bevorzugt Natrium- oder Kaliumpyruvat, ist.

10. Verfahren nach einem der Ansprüche 1 bis 9, wobei das Salz ein ¹³C-angereichertes Pyruvat ist, welches an der C1-, C2- oder C3-Position Isotopen-angereichert ist.

11. Verfahren nach Anspruch 10, wobei das ¹³C-angereicherte Pyruvat durch Metallisierung von 2-Methyl-1,3-dithian oder 2-Methyl-1,3-dithian, welches an der Methylgruppe ¹³C-angereichert ist, synthetisiert wird und welches mit wahlweise ¹³C-angereichertem CO₂ reagiert, wobei zumindest das CO₂ oder das 2-Methyl-1,3-dithian eine ¹³C-angereicherte Verbindung ist und die Carbonyl-Funktion freisetzt.

12. Verfahren nach Anspruch 11, wobei das ¹³C-angereicherte Pyruvat ein an der C1-Position angereichertes ¹³C-angereichertes Pyruvat ist, welches durch Metallisierung von 2-Methyl-1,3-dithian synthetisiert wird und welches mit ¹³C-angereichertem CO₂ reagiert und die Carbonyl-Funktion freisetzt.

13. Verfahren nach Anspruch 10, wobei das ¹³C-angereicherte Pyruvat durch Umsetzung von wahlweise ¹³C-angereicherter Essigsäure in Acetylbromid synthetisiert wird und das Acetylbromid mit wahlweise ¹³C-angereichertem CuCN umgesetzt wird, wobei zumindest die Essigsäure oder das CuCN eine ¹³C-angereicherte Verbindung ist, um ¹³C-angereichertes Pyruvamid zu erhalten, welches dann hydrolysiert wird.

14. Verfahren nach Anspruch 10, wobei das ¹³C-angereicherte Pyruvat ein an der C1-Position angereichertes ¹³C-angereichertes Pyruvat ist, welches durch Umsetzung Essigsäure in Acetylbromid synthetisiert wird und das Acetylbromid mit ¹³C-angereichertem CuCN umgesetzt wird, um ¹³C-angereichertes Pyruvamid zu erhalten, welches dann hydrolysiert wird.

## Revendications

1. Procédé de production d'acide pyruvique comprenant la réaction d'un sel d'acide pyruvique avec de l'acide chlorhydrique en la présence d'un solvant ou mélange de solvant à partir d'un groupe constitué par des nitriles et cétones polaires aprotiques et, isolation de l'acide pyruvique brut obtenu et, en variante, la purification dudit acide pyruvique brut.

2. Procédé selon la revendication 1 dans lequel le solvant est de l'acétone, de l'acétylacétone, de la méthyléthylcétone, de la méthylisobutylcétone, de l'acétonitrile ou du valéronitrile ou un mélange de ceux-ci.

3. Procédé selon les revendications 1 à 2, dans lequel le solvant est de l'acétone ou de l'acétonitrile ou un mélange de ceux-ci.

4. Procédé selon les revendications 1 à 3, dans lequel l'acide pyruvique brut est isolé par filtrage du sel résultant de la réaction et/ou de l'évaporation du solvant ou du mélange de solvant utilisé.

5. Procédé selon les revendications 1 à 4, dans lequel l'acide pyruvique brut est purifié.

6. Procédé selon la revendication 5, dans lequel la purification est mise en oeuvre par l'extraction de l'acide pyruvique, suivie, en variante, par une distillation de l'acide pyruvique purifié.

7. Procédé selon la revendication 5, dans lequel la purification est mise en oeuvre par précipitation du sel restant dans l'acide pyruvique brut, suivie, en variante, par une distillation de l'acide pyruvique purifié.

8. Procédé selon la revendication 7, dans lequel ladite précipitation est favorisée par ajout d'un solvant ou mélange de solvant à partir du groupe constitué par un éther, un ester ou une cétone, de préférence un éther et une cétone, plus préférablement de l'acétone.

9. Procédé selon les revendications 1 à 8, dans lequel ledit sel d'acide pyruvique est un sel de métal alcalin ou un sel de métal alcalino-terreux, de préférence du pyruvate de sodium ou de potassium.

10. Procédé selon les revendications 1 à 9, dans lequel ledit sel est un pyruvate enrichi en 13C qui est enrichi de manière isotopique à la position C1, C2 ou C3.

11. Procédé selon la revendication 10, dans lequel le pyruvate enrichi en 13C est synthétisé par métallation de 2-méthyle-1,3-dithian ou de 2-méthyle-1,3-dithian qui est enrichi en 13C au niveau du groupe méthyle et réaction avec du CO2, en variante, enrichi en 13C, dans lequel au moins le CO2 ou le 2-méthyle-1,3-dithian est un composé enrichi en 13C, et libération de la fonction carbonyle.

12. Procédé selon la revendication 11, dans lequel le pyruvate enrichi en 13C est un pyruvate à base de 13C enrichi à la position C1 qui est synthétisé par métallation de 2-méthyle-1,3-dithian, réaction avec du CO2 enrichi en 13C et libération de la fonction carbonyle.

13. Procédé selon la revendication 10, dans lequel le pyruvate enrichi en 13C est, en variante, synthétisé par conversion d'acide acétique enrichi en 13C en bromure d'acétyle, réaction du bromure d'acétyle avec, en variante, du CuCN enrichi en 13C, dans lequel au moins l'acide acétique ou le CuCN est un composé enrichi en 13C afin d'obtenir du pyruvamide enrichi en 13C qui est alors hydrolysé.

14. Procédé selon la revendication 10, dans lequel le pyruvate enrichi en 13C est un pyruvate à base de 13C enrichi à la position C1 qui est synthétisé par conversion d'acide acétique en bromure d'acétyle, réaction du bromure d'acétyle avec du CuCN enrichi en 13C afin d'obtenir du pyruvamide à base de 13C1 qui est ensuite hydrolysé.
